# EUROPEAN PATENT SPECIFICATION

(11) **EP 1 718 359 B1**
(45) Date of publication and mention of the grant of the patent: **23.11.2011**
(21) Application number: 05722861.1
(22) Date of filing: 10.02.2005
(51) Int. Cl.: A61M 31/00

(54) **INTRAVASCULAR DELIVERY SYSTEM FOR THERAPEUTIC AGENTS**
INTRAVASKULÄRES ABGABESYSTEM FÜR THERAPEUTISCHE MITTEL
SYSTEME D'ADMINISTRATION INTRAVASCULAIRE D'AGENTS THERAPEUTIQUES

(30) Priority: 10.02.2004 US 543260 P; 09.12.2004 US 634585 P
(43) Date of publication of application: 08.11.2006
(73) Proprietor: Synecor, LLC, Durham, NC 27703 (US)
(72) Inventor: WILLIAMS, Michael, S., Santa Rosa, CA 95404 (US); HOLBROOK, Kevin, Chapel Hill, NC 27516 (US); RANSBURY, Terrance, Chapel Hill, NC 27517 (US)
(74) Representative: Jackson, David Spence
(86) International application number: PCT/US2005/004063
(87) International publication number: WO 2005/077450

(56) References cited:
- US-A- 4 900 303
- US-A- 5 368 588
- US-A- 5 372 585

## Description

The present invention relates to a system for drug delivery, the system comprising an implantable reservoir and an implantable elongate device body.

Numerous drugs cannot be taken orally for various reasons. For example, oral ingestion of certain agents cannot be tolerated by the GI systems of some patients, or will result in severe systemic side effects. Other agents cannot withstand a gastrointestinal route of administration without breaking down and becoming ineffective. Some agents must be targeted to specific organs or tissues and thus are not suitable for oral ingestion. It would thus be highly desirable to administer these drugs, including drugs that are conventionally delivered intravenously, using an automated implanted administration system.

An automated implantable administration system can also benefit patients taking drugs that generally can be taken orally. For example, oral administration can be impractical in patients who are unable to self-administer a required dosage when needed. Administration using an automated implantable administration system is further beneficial in that it can administer a drug when a physical or chemical condition is detected by the system's sensor (e.g. reduced blood sugar), but before the patient suffers from the imminent symptoms. An automated system can also accelerate the desired systemic or local response to the drug administration by eliminating the time necessary for an orally ingested drug to be absorbed from the stomach into the bloodstream. Also, a lower dosage of a drug may be used (e.g. in some cases as little as 1% of the dosage needed for oral ingestion) when the drug is administered directly into the bloodstream. Finally, an automated system also allowed for automatic dosage modification based on detected patient conditions.

In view of the foregoing need, a system has been developed which is fully or partially positionable within the vascular system of a body, and which can deliver pharmacological agents to induce a therapeutic effect. The present invention provides an implantable intravascular drug delivery system, which allows administration of therapeutic agents ("or drugs") directly into the vasculature.

US 5368588 describes a system of the kind defined hereinbefore at the beginning in which the reservoir, or its outer wall, is made of a shrink polymer which is actuated, and therefore contracts, in response to the body temperature of a patient in which it is implanted. The elongate body is a small diameter tube connected to the reservoir and containing alternating changes of non-miscible medications, the contracting reservoir acting to pump out the medications.

According to the present invention, a system of the kind defined hereinbefore is **characterised in that** the reservoir and the device body are proportioned for implantation within a blood vessel, the elongate device body being flexible and having an anchor coupled thereto and expandable into contact with a wall of the blood vessel; and a pump being housed within the device body and fluidly coupled to the reservoir and operable to direct agent from the reservoir into the bloodstream.

The invention will now be described by way of example with reference to the accompanying drawings, in which:
Fig. 1 is a front perspective view showing a first embodiment of an intravascular drug delivery system.
Fig. 2 is a perspective view of a housing segment of the first embodiment, illustrating features of a gear pump housed within the segment.
Fig. 3A is a perspective view similar to Fig. 2 showing a peristaltic pump which may be used as an alternative to the pump of Fig. 2. Fig. 3B is a cross-sectional end-view of the housing segment further illustrating features of the Fig. 3A pump.
Figs. 4 through 6A are side elevation views of other alternative pump designs. In these views, the pump chamber is shown in cross-section to allow the interior components to be seen. Fig. 6B is a perspective view of a device incorporating the pump of Fig. 6A.
Fig. 7 is a side elevation view of a second embodiment of an intravascular drug delivery system.
Fig. 8A is a perspective view of an anchor suitable for use with implantable drug delivery systems.
Fig. 8B is a perspective view showing the anchor of Fig. 8A attached to an implantable electrophysiological device and in the expanded position.
Fig. 8C is a cross-sectional end view of the device shown in Fig. 8B.
Fig. 8D is a side elevation view of a device showing the anchor of Fig. 8B positioned on a device and compressed by a sheath.
Fig. 8E is similar to Fig. 8D but shows retraction of the sheath to permit expansion of the anchor within a blood vessel.
Figs. 9A through 9C are a sequence of drawings schematically illustrating implantation of the device of Fig. 1 within the vasculature.
Fig. 9D illustrates a third embodiment of an intravascular drug delivery device positioned within the vasculature.
Fig. 9E illustrates a fourth embodiment of an intravascular drug delivery device positioned within the vasculature, and with the fill tube of the device withdrawn from the body for use in refilling the device reservoir.
Fig. 10 is a front elevation view showing a fourth embodiment of an intravascular drug delivery device having delivery conduits for localized drug delivery.
Fig. 11 schematically illustrates an intravascular drug delivery device anchored within a blood vessel and having a delivery conduit positioned in the hepatic artery.
Fig. 12 is similar to Fig. 11 but shows the device positioned in the descending aorta and delivery conduits positioned in the renal arteries.
Fig. 13 schematically illustrates positioning of an intravascular drug delivery device which includes a refill port disposed in the right axillary vein.

### Detailed Description

This application describes fully or partially intravascular systems for administering drugs including hormones, chemotherapeutic agents, antibiotics pharmaceuticals, synthetic, recombinant or natural biologics, and other agents within the body. Generally speaking, the systems include drug reservoirs and associated components that are anchored in the vasculature and that administer drugs into the bloodstream or into certain organs or tissues. Throughout this disclosure, the terms "drugs" and "agent" will be used to refer to substances to be delivered into the body using systems of the type described herein.

In some embodiments, the systems may be programmed to deliver agents into the body according to a particular delivery schedule. For example, the device may be programmed to deliver 10 cc of agent over a 24-hour period every two weeks. According to this example, the device's control system may be programmed to activate the pump continuously at a specified rate during the 24-hour period. Alternatively, the control system may be programmed to cause the pump to deliver a single bolus of the agent every minute over the 24-hour period. This latter algorithm can extend battery life by avoiding continuous draw on the battery (e.g., by the pump or associated motor) for an extended duration. Delivery schedules may be set and/or adjusted using an external programming device (e.g., a handheld device, PC, or other microprocessor device) that communicates with the implantable device using radio frequency encoded signals or other telemetric methods.

In other embodiments, the systems may be controlled via internal intelligence that is responsive to in-situ diagnostic analysis of liquid, chemical or physical changes in the patient. Such systems can utilize feedback from sensors on the implantable device to initiate release of the agent. The internal intelligence may be provided with various levels of sophistication. For example, the device may be programmed to simply deliver a prespecified volume of agent when a detected parameter exceeds a specified level; or it may be equipped to select the volume of the agent to be delivered depending on the severity of the change in detected parameters and/or the amount of time elapsed since the last administration of the agent.

Alternatively, the systems may be configured to deliver agents "on-demand" when prompted by a patient to do so. In this type of system, patient communication may be carried out using an external programming device, or using remote activators that use magnetic, radio frequency, infrared, acoustic, or other triggers to initiate drug delivery.

### Applications

Systems of the type described herein find use in many areas of medicine. Applications for the present technology include, but are not limited, to the following:

### Cardiovascular Applications:

An intravascular drug delivery system may be used to treat cardiovascular conditions and/or their symptoms by delivering suitable agents into the blood within the vasculature and/or the heart. For example, the system may be used to deliver agents used to treat symptoms of congestive heart failure (CHF). Such agents may include agents within the classes of positive inotropes, diuretics, vasodilators, and cytokine effectors. Specific agents include: Dobutamine, Atrial Natriuretic Peptide, Digoxin, Enoximone, Nesiritide, Tezosentan, Bumetanide, Hydralazine, Alprostadil, Carvedilol, Enalaprilat, Ambrisentan, and Levosimendan (sold by Abbott Laboratories under the trade name Simdax)

As discussed, the drug may be administered according to a pre-programmed delivery protocol (for example X volume every Y seconds for a period of Z days), or in response to telemetric instructions provided by a physician or patient, or in response to closed-loop feedback from a sensor forming part of the system. Such sensors might be positioned on or coupled to the implantable system, or they may communicate with the system from a remote location elsewhere in the body.

Thus a system for treating CHF symptoms might include a sensor for detecting conditions indicative of CHF. The sensor may be of a type to detect any of a number of criteria including but not limited to: arterial pressure, such as in the right atrium, right ventricle, and/or pulmonary artery; cardiac output; heart rate; Q-T interval; AVO₂ difference; blood pH (including as an indicator of lactic acid levels in the blood); blood gas levels (including blood 0₂ and/or blood CO₂ levels).

The system might also include a sensor for detecting biochemical markers which might include:
(1) Triage Cardiac - a unique set of three biochemical indicators of cardiac muscle necrosis: Mioglobine; CK-MB and Cardiac Troponine I. Alternatively, the system may rely on one or two of these markers alone or in combination with other markers.
(2) Brain Natriuretic Peptide - a non-invasive, objective marker of Congestive Heart Failure. Research indicates that
   - The concentration of BNP increases with the severity of CHF (precise correlation with NYHA classification).
   - BNP concentration has the positive correlation with end-diastolic pressure in left ventricle.
   - There is a reverse ratio between BNP level and the function of left ventricle after heart infarction.
   - The increase of BNP level is associated with increasing of Pulmonary Artery Wedge Pressure (precise correlation), deterioration of LV diastolic and systolic functions, LV Hypertrophy and Heart Infarction.
(3) Tumor Necrosis Factor. Elevated levels of the immune factor tumor necrosis factor (TNFa) may be very strong and accurate predictors of a poor outlook in CHF patients. This immune factor is known to be a potent agent in the inflammatory process.

In one example of a closed-loop type system, the sensor might be a pH sensor for detecting blood acid levels, since a patient suffering from congestive heart failure (CHF) typically possesses elevated levels of lactic acid in his/her bloodstream. According to this embodiment, a delivery system might be programmed to deliver Dobutamine or another agent in response to detection of elevated lactate levels.

### Diabetes

An intravascular drug delivery system may also be configured to deliver insulin to diabetic patients. According to one embodiment, an intravascular insulin-delivery system may be a closed-loop system including a glucose sensor for measuring blood sugar levels and an insulin reservoir. This embodiment of the system may be programmed to administer appropriate doses of insulin as needed by the patient.

### Emphysema and Asthma

An intravascular system may include an O₂ or CO₂ sensor equipped to detect hypoxia or hypercarbia in the patient's blood. In response, the system may administer a bronchodilator and/or other medications such as NO (nitric oxide) at the earliest onset of hypoxemia or hypercarbia, even before the patient becomes aware of the onset of the condition.

### Organ Specific Examples

By directing drugs to a particular aortic branch (e.g., hepatic artery, renal artery, etc), an intravascular delivery device can achieve target delivery of therapeutic drugs (including chemotherapy, gene therapy or other organ-specific therapeutics) to specific organs including the brain, liver, kidneys, pancreas, lung, etc. For example, drugs may be directed towards the brain to treat diseases such as Alzheimer's, Parkinson's, or epilepsy; towards the brain, liver, kidneys, pancreas or lungs for cancer treatment; or towards the kidneys to treat cardio-renal syndrome that can be associated with congestive heart failure. Drugs may be directed towards the lungs via the venous system for treatment of conditions such as asthma or pulmonary hypertension, or via the arterial system for treatment of lung cancer.

The system may also be used to deliver drugs or chemicals to a specific organ in order to enhance the sensitivity of the tissues in that organ to externally or internally delivered therapies such as radiation.

### Cancer

Another embodiment of an intravascular system could administer chemotherapy according to a pre-programmed timetable, or in response to telemetric instructions received from a physician. In some embodiments, the system may be positioned for targeted delivery of agents into blood vessels that feed vascularized tumor masses. The system may also be used to deliver radioactive particles to target sites.

### Chronic Pain Management

Many patients suffering from chronic pain are candidates for PCA (Patient Controlled Analgesic), which is presently administered intravenously in hospitals. An intravascular system of the type described here would allow patients to control pain using PCA while remaining ambulatory.

### Other Examples

Other applications for intravascular drug delivery systems include treatment of ophthalmic conditions, blood conditions such as hemophilia, as well as diseases and conditions not specifically mentioned herein.

### System

Generally speaking, an intravascular drug delivery system may include a variety of components, the selection of which will vary depending on the application for the device and its intended drug delivery protocol (i.e., closed-loop vs. programmed delivery protocol vs. on-demand or telemetric initiation of delivery).

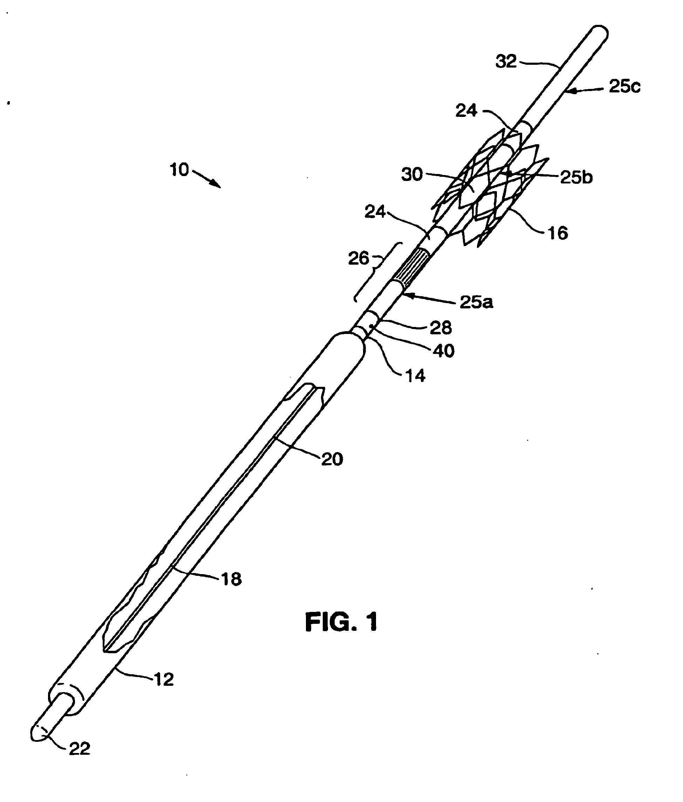

A first embodiment of an intravascular drug delivery device 10 is a fully intravascular system as shown in Fig. 1. The device of the first embodiment is preferably programmed to deliver an agent according to a time schedule, although it may be modified to administer drugs on-demand or in response to sensor feedback as described elsewhere in this application.

Device 10 includes a drug reservoir 12, a device body 14, and a retention device or anchor 16 for retaining the device 10 within the vasculature.

The reservoir 12 may take the form of a flexible inflatable bladder at least partially formed of a thin membrane. The bladder may be implanted prior to inflation, and then filled the necessary agent once implanted. The bladder might be formed of polyurethane, polyethylene or similar materials capable of withstanding rupture and degradation during implantation and use, and suitable for containing the agents to be delivered. Although the bladder is shown as having a cylindrical shape, other shapes (e.g., a crescent shape) may be selected so as to reduce the overall length of the device and/or to reduce the cross-sectional profile of the device at certain points along its length. In one embodiment, the reservoir may have a volume of approximately 40 ml, although larger or smaller reservoirs will be used when warranted by the concentration of the agent and the number and size of anticipated doses.

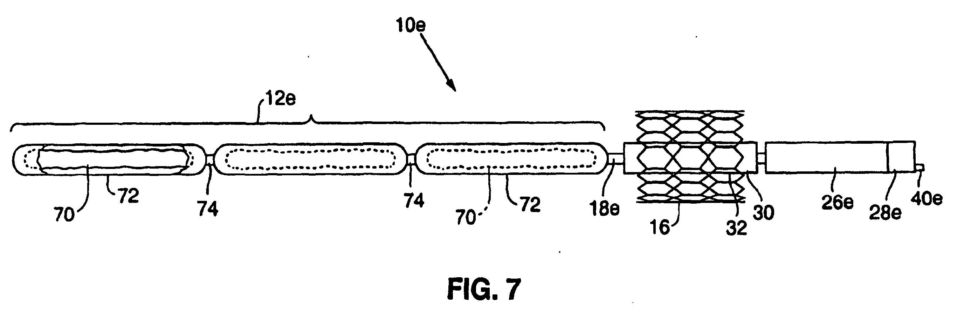

An alternative reservoir embodiment might include a non-inflatable reservoir formed of titanium or suitable polymeric materials. As yet another alternative (which is described in connection with Fig. 7), the reservoir may take the form of one or more inflatable bladders housed within one or more protective enclosures formed of titanium, polymeric material, or other suitable materials.

The reservoir is proportioned to minimize obstruction of blood flow even when inflated. The cross-sectional area of the reservoir in the transverse direction (i.e., transecting the longitudinal axis) should be as small as possible while still accommodating the required volume of drug. This area is preferably in the range of approximately 79 mm² or less, and more preferably in the range of approximately 40 ^{mm2} or less, or most preferably between 12.5 - 40 ^{mm2}. For a cylindrical reservoir, a cross-sectional diameter in the range of approximately 3 - 15 mm may be suitable.

An elongate pickup tube 18 extends through the reservoir. The tube is preferably manufactured of a material having sufficient flexibility to permit flexing of the tube as the device passes through bends in the patient's blood vessels, but also having sufficient kink-resistance to prevent kinks from forming in the tube during bending. The material should also be one that will not corrode or degrade in the presence of the agent to be delivered. Nitinol and suitable polymeric materials are examples.

The walls of the tube 18 include one or more openings 20. During use, the agent is drawn into the tube 18 via these openings. The agent flows through the tube 18 into a pump chamber from which it may then be pumped from the pump chamber into the bloodstream.

A reservoir fill port 22 is fluidly coupled to the reservoir 12 and is configured to receive a needle or other device that may to fill and/or re-fill the reservoir 12. The port 22 may also be engaged by an implantation tool which can be used to push the device 10 through the vasculature and into the desired location within the body.

Device body 14 houses various components used to carry out drug delivery. The components within the device are disposed within an enclosure that is a rigid, semi-rigid or flexible housing preferably formed of a material that is biocompatible, capable of sterilization and capable of hermetically sealing the components contained within it. Various materials may be used for the enclosure, including molded compounds, metals such as titanium or stainless steel, or other materials. The exterior of the enclosure may be anti-thrombogenic (e.g., ePTFE or perfluorocarbon coatings applied using supercritical carbon dioxide) so as to prevent thrombus formation on the device. It may also be beneficial that the coating have anti-proliferative properties so as to minimize endothelialization or cellular ingrowth, since minimizing growth into or onto the device will help minimize vascular trauma when the device is explanted. The coating may thus also be one which elutes anti-thrombogenic compositions (e.g., heparin sulfate) and/or compositions that inhibit cellular ingrowth and/or immunosuppressive agents. Coatings of a type that may be used on the device 10 include those described in U.S. Application No. 11/020,779, filed December 22, 2004, entitled Liquid Perfluoropolymers and Medical Applications Incorporating Same. Others include nanocoatings provided by Nanosys of Palo Alto, CA.

Alternatively, the housing exterior may include a coating or surface that functions as a tissue ingrowth promoter, thus aiding in retention of the device within the vasculature. As yet another example, the device 10 may be insertable into a separately implantable flexible "exoskeleton" that is pre-implanted into the vasculature. The exoskeleton includes a pocket into which the device 10 is inserted. Eventually, anchoring of the exoskeleton within the vessel may become reinforced by tissue ingrowth, whereas the device 10 remains free of ingrowth. This allows the device to be withdrawn from the exoskeleton, leaving the exoskeleton in place with minimal trauma to the vessel walls. This would facilitate removal for various purposes, including refilling or replacement of fluid reservoirs, replacement of batteries, replacement of the device with a fresh device, or for other purposes. The original or replacement device might then be passed into the vasculature and inserted into the exoskeleton. Examples of exoskeleton configurations are described in U.S. Application No. 11/009,649, filed December 10, 2004, entitled IMPLANTABLE MEDICAL DEVICE HAVING PRE-IMPLANT EXOSKELETON

The device is proportioned to be passed into the patient's vasculature and to be anchored within the vasculature with minimal obstruction to blood flow. Suitable sites for the device 10 may include, but are not limited to the venous system using access through the right or left femoral vein or the subclavian or brachiocephalic veins, or the arterial system using access through one of the femoral arteries. Thus, the housing 14 preferably has a streamlined maximum cross sectional diameter which may be in the range of 3 - 15 mm or less, with a most preferred maximum cross-sectional diameter of 3-8 mm or less. The cross-sectional area of the device in the transverse direction (i.e., transecting the longitudinal axis) should be as small as possible while still accommodating the required components. This area is preferably in the range of approximately 79 mm² or less, and more preferably in the range of approximately 40 mm² or less, or most preferably between 12.5 - 40 mm².

The cross-section of the device (transecting the longitudinal axis) may have a circular cross-section, although other cross-sections including crescent, flattened, or elliptical cross-sections may also be used. It is desirable to provide the device with a smooth continuous contour so as to avoid voids or recesses that could encourage thrombus formation on the device.

Depending on the length of the device, it may be advantageous to manufacture flexibility into the housing so that it can be easily passed through the vasculature. In the Fig. 1 embodiment, the device body is divided into housing segments 25, each separated by flexible articulations 24 which may be formed using silicone rubber filler or other material. The articulations 24 form living hinges, which bend in response to passage of the device 10 though curved regions of the vasculature. For some embodiments, which may have device bodies of approximately 10 - 60 cm in length (including the length of the reservoir) with individual segments ranging from approximately 2 - 28 cm in length, flexibility of the device may be essential for movement and positioning of the device within the vasculature with minimal damage to the blood vessels.

Alternatively, flexibility may be achieved through the use of flexible materials for the device body.

A motor 26, pump 28, control circuitry and electronics 30, and a battery 32 for powering operation of the motor and electronics are housed within the device body 14. Although a particular arrangement of components is shown in Fig. 1, the components may be arranged in a variety of different ways, although it is desirable to arrange the components so as to make efficient use of the available space so as to minimize unnecessary bulk or length.

These components may be contained within separate housing segments 25a, 25b, 25c, in which case electrical conductors may extend through the articulations 24 as needed to electrically couple the components in each of the housing segments. The mechanical elements used to connect the housing segments 25a, 25b, 25c are preferably designed such that axial, flexural and torsional forces imparted to the device are transmitted by the mechanical elements rather than by the electrical conductors that extend between the segments to electrically couple the various components of the device. Suitable arrangements of mechanical and electrical elements for coupling between housing segments are described in U.S. Application No. 10/862,113, filed June 4, 2004, and entitled INTRA VASCULAR ELECTROPHYSIOLOGICAL SYSTEM AND METHODS.

Battery 32 may be a 3 V lithium battery although other batteries suitable for the parameters of the motor and proportioned to fit within the housing may also be used.

The electronics 30 include control circuitry for controlling operation of the motor or other pumping mechanisms. If a closed-loop system is employed, the electronics 30 will also include intelligence for receiving data concerning parameters detected by sensors and for initiating delivery of an appropriate quantity of the agent. The electronics 30 may also include telemetry circuitry allowing for on-demand control of delivery, and dosage programming and/or modification.

Various dispensing mechanisms may be used to move or pump the agent from the reservoir into the blood stream. These mechanisms include but are not limited to:
- diffusion of the agent through a polymeric membrane;
- osmotic pumps (e.g., pumps of the type sold by ALZA Scientific products under the trade name ALZET) which pump via osmotic pressure induced by movement of fluid into the reservoir (i.e., by osmosis) and exertion of force on a dynamic membrane which pushes the agent out of the reservoir and into blood, tissues or organs);
- electro-mechanical micropumps. Examples include gear pumps, solenoid pumps, peristaltic pumps, vacuum pumps, venturi pumps, double-acting membrane pumps, syringe pumps and vaporization displacement pumps.
- a controlled-rate compression membrane within a reservoir housing which drives the agent through an exit orifice in the reservoir at a specific rate. A variety of mechanisms may be used to cause the compression membrane to bear against the fluid in the reservoir. Such mechanisms include rechargeable osmotic pumps, compressive sleeves, or expandable springs or sleeves;
- ameroid constrictor-type control, in which a ring having an adjustable orifice is disposed around a compliant reservoir and in which the inner diameter of the ring is increased/decreased to increase/reduce flow of the agent

In another embodiment of an intravascular delivery device, the device may include a plurality of tiny agent-containing reservoirs, each sealed by a barrier layer (e.g., a polymer or a low-melt metal or allow such as gold). According to this embodiment, delivery of the agent is achieved through the application of energy (such as RF, ultrasound, or other energy forms) to the barrier layer of one or more the reservoirs. The energy induces micro-erosion of the barrier, allowing the agent to be diffused or pumped to the bloodstream or to a target site. This may be achieved through the use of MEMS technologies, which allow mechanical elements and electronics to be formed on tiny sections of silicon substrate.

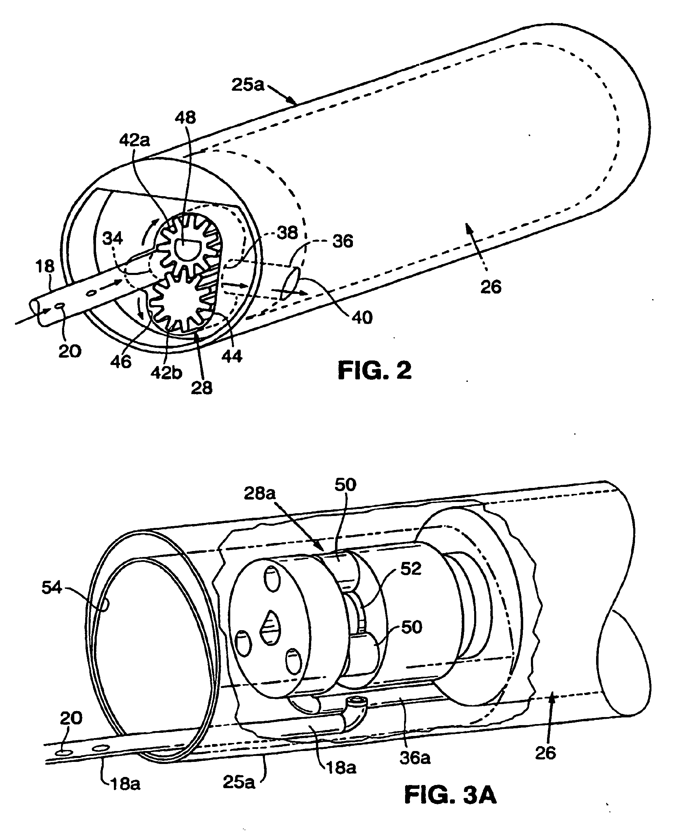

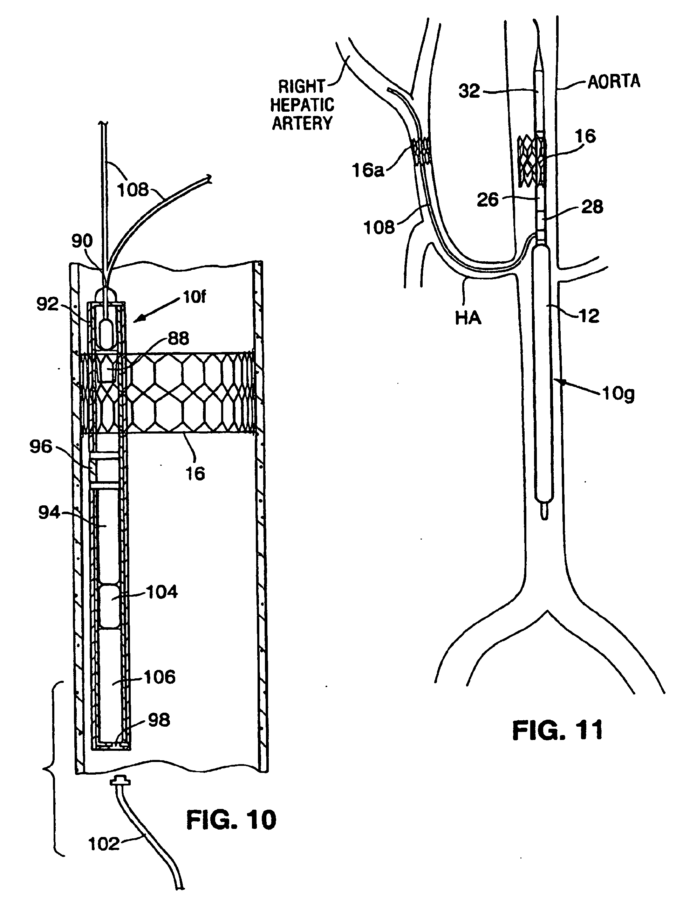

In the Fig. 1 embodiment, a pump is preferably used to pump agent into the bloodstream. One type of a pump particularly useful in the device 10 is a gear pump which propels fluid using a pair of rotating gears. Referring to Fig. 2, the pump 28 and motor 26 are disposed within one of the housing segments 25a forming the device body. Pickup tube 18, which extends through reservoir 12 (see Fig. 1), has an outlet 34 (Fig. 2) positioned within the housing segment 25a. An exit tube 36 has an inlet port 38 within the segment 25a and extends to the exterior face of the housing segment wall to form a delivery port 40 through which drug is released from the device. One or more such delivery ports may open directly into the bloodstream. The exit tube 36 may be adjacent to the pump as shown, or it may extend to a remote portion of the device, such as the distal end of the device. Drug release may be antegrade to blood flow, or it may be retrograde so as to promote mixing of the drug with the blood. If more localized delivery of agent is desired, the device may include delivery conduits of the type described below in connection with Figs. 10-12.

Pump 28 includes a pair of gears 42a, 42b disposed between the outlet 34 of the pickup tube 18 and the inlet port 38 of the exit tube 36. The teeth of the gears are enmeshed such that rotation of one gear will compel rotation of the other gear. A fluid impermeable barrier 44 surrounds the outlet 34, gears 42a, 42b and inlet port 38 to form a pump chamber 46. The barrier 44 is spaced slightly from the outermost points of the gears so as to permit rotation of the gears.

Gear 42a includes a socket 48 for receiving a shaft (not shown) that is driven by the motor. Thus, activation of the motor drives the gear 42a, which in turn causes gear 42b to rotate.

During operation, rotation of the gears in the direction of the arrows shown in Fig. 2 causes fluid to be drawn from the reservoir 12 (Fig. 1) through the pickup tube 18 and into the pump chamber 46 (Fig. 2). The fluid is propelled into and through the exit tube 36 by the teeth of the gears, thus causing the agent to pass through exit port 40 and into the bloodstream.

Figs. 3A through 6 illustrate alternative pump configurations that may be used in an intravascular drug delivery system.

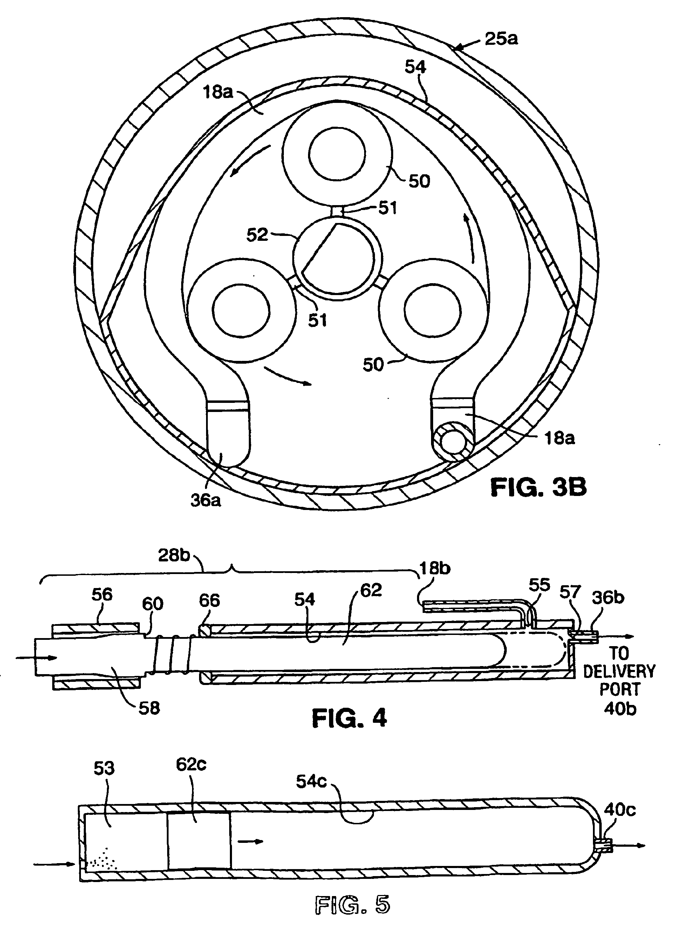

In one alternative embodiment, the pump may take the form of a peristaltic pump. Referring to Figs. 3A and 3B, the peristaltic pump 28a includes a plurality of rollers 50 coupled to a central hub 52 by radially-extending arms 51 (Fig. 3B). Motor 26 (Fig. 3A) is coupled to the hub such that activation of the motor rotates the hub 52, causing the rollers 50 to revolve around the hub.

In this embodiment, pump pick-up tube 18a extends from the reservoir 12 (Fig. 1) into the housing segment 25a, and extends around the rollers 50 as shown in Fig. 3B. (It should be noted that the portion of the tube 18a that extends around the bearings 50 has been omitted from Fig. 3A for clarity.) Pump pick-up tube 18a is fluidly coupled to an exit tube 36a that is in turn coupled to an exit port. Although not shown in Figs. 3A and 3B, the exit port may be similar to exit port 40 of Fig. 1 or it may be positioned in a more remote location on the device body.

As best shown in Fig. 3B, a wall 54 at least partially surrounds the rollers 50. At least a portion of the wall is positioned sufficiently close to the roller/hub assembly to cause a portion of the pick-up tube 18a to be squeezed against the wall 54 by the rollers 50 during rotation of the hub 52. In other words, a portion of the wall 54 is positioned such that activation of the motor 26 causes rollers 50 to roll over, and thus temporarily compress, the tube 18a against the wall. It can be seen from Fig. 3B that the tube becomes squeezed against the upper-most portion of wall 54 by the passing roller bearing. The temporary compression of the tube 18a drives fluid in the tube out the exit tube 36a to the exit port. When a roller 50 separates from the delivery tube 18a, compression is released, thereby creating suction within the pick-up tube 18a to draw additional fluid into it from the reservoir. Continued rotation of the hub will cause the rollers to sequentially squeeze and release the tube until the motor is turned off. Naturally, the portion of the tube 18a that extends around the rollers is preferably formed of a flexible and compressible material such as an appropriate polymer.

Another alternative type of pump 28b is shown in Fig. 4. Pump 28b is a displacement type pump which uses a solenoid-driven plunger to drive fluid from a fluid chamber. This embodiment eliminates the need for the motor shown in prior embodiments.

For simplicity, the housing segment (such as segment 25a in Fig. 1) surrounding the pump is not shown in Fig. 4. Referring to the figure, the pump 28b includes a chamber 54 fluidly coupled to pickup tube 18b. As with prior pump examples, the pickup tube 18b is fluidly coupled to a reservoir containing the agent to be delivered to the patient. A one-way valve 55 within the pickup tube 18b permits fluid flow into, but not out of, the chamber 54. An exit tube 36b extending from the chamber 54 provides a flow path for movement of agent to a delivery port 40a/. The delivery port may alternatively be formed directly into the chamber 54. The exit tube 36b or delivery port 40b includes a one-way valve 57 that allows only outward flow of fluid from the chamber 54.

A solenoid 56 is positioned adjacent to the chamber 54. Energy for the solenoid is provided by the battery 32 (Fig. 1). Referring again to Fig. 4, a magnetic piston 58 is slidable within the solenoid 56. Piston 58 includes a broad section that tapers at a shoulder 60 to form a plunger 62. A portion of the plunger 62 extends through an opening in the chamber 54. A seal 66 preferably surrounds the opening to prevent leakage of fluid from the chamber 54. It should be noted that the solenoid might instead be sealed within the chamber 54 to prevent loss of agent from the chamber.

The plunger 62 moves between the retracted position shown in solid lines in Fig. 4, and the extended position shown in dashed lines. A spring 64 sits between the piston's shoulder 60 and the wall of chamber 54, and functions to bias the plunger 62 in the retracted position.

To pre-load the pump chamber with agent, the solenoid 56 is energized to drive the piston 58 forward, causing the plunger 62 to advance within the chamber. The chamber 54 may come preloaded with a volume of saline to prevent this step from expelling air through delivery port 40 into the bloodstream. The shoulder 60 of the advancing piston compresses the spring 64 such that, upon de-energization of the solenoid, the spring expands against the shoulder 60 to return the plunger to the retracted position. Retraction of the plunger causes the chamber to fill with agent, by creating a vacuum which draws agent into the chamber via pickup tube 18b. Naturally, the chamber 54 and the plunger 58 are proportioned such that advancement of the plunger dispenses a volume of the agent that is appropriate for the patient's condition.

To deliver the agent to the patient, the solenoid is energized, causing the plunger to drive the agent out through the delivery port 40b. Once the agent has been delivered, the solenoid is de-activated, causing the plunger 62 to retract and to draw additional agent into the chamber 54 via pickup tube 18b.

It should be appreciated that the Fig. 4 embodiment may be modified to use alternative mechanisms for advancing and retracting the plunger. For example, a motor may provided with a lead screw that is coupled to the plunger. In this example, operation of the motor at a first polarity causes advancement of the plunger, whereas operation of the motor at a reverse polarity causes retraction of the plunger. As another example shown in Fig. 5, the plunger 62c may be advanced by fluid (e.g., saline) introduced into a section 53 of the chamber 54c as indicated by arrow A. The pressure of the introduced fluid advances the plunger within the chamber 54c, thereby driving the agent out the delivery port 40c. The fluid may be introduced directly into the chamber as shown, or in may be introduced into a bladder that expands against the plunger 62c.

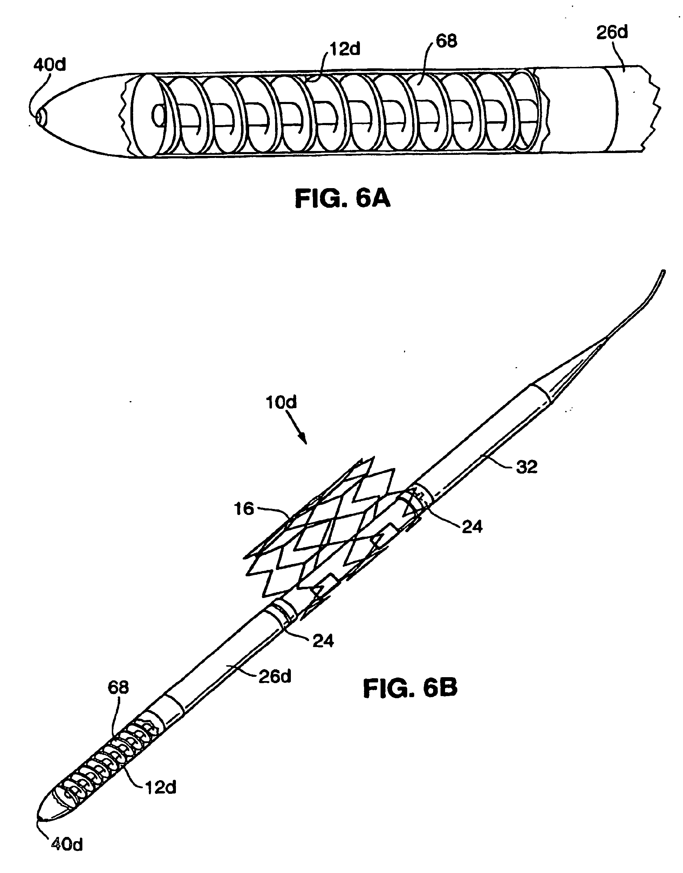

Fig. 6A shows yet another pump configuration using a metering screw 68 positioned within a chamber containing the agent. In this embodiment as well as the others, the chamber may be the agent reservoir 12d itself, or it may be a separate chamber fluidly coupled to a fluid reservoir as described in previous embodiments. Rotation of the metering screw 68 causes the threads of the screw to push agent in the reservoir towards a delivery port 40d. During use, the metering screw 68 is rotated for an appropriate number of turns to cause the necessary amount of drug to be dispensed through a delivery port 40d. The metering screw 68 is rotated using motor 26d, which may be a stepper motor to facilitate precise metering of the agent. Fig. 6B illustrates the pump configuration of Fig. 6A on a device 10d having features similar to those shown in Fig. 1. The device 10d additional includes an atraumatic distal end including a wire 11 to facilitate guidance of the device within a blood vessel. If the device 10d includes telemetry circuitry, wire 11 may also be used for telemetric communication to a remote communications device.

Fig. 7 shows an alternative embodiment of an implantable drug delivery device 10e which differs from the Fig. 1 embodiment largely in the construction of the reservoir. Referring to Fig. 7, reservoir 12e is formed of a bladder 70 (e.g., polyurethane, polyethylene or similar material) housed within a durable housing 72. The housing is preferably formed of titanium, molded compounds, or other durable and biocompatible materials. If the compound to be housed in the reservoir 12e is thermally sensitive, the reservoir may include insulating materials such as to isolate the agent against the warming effects of the surrounding blood. If necessary, the housing may be equipped with a cooling system for maintaining the temperature of the agent within a desired range. Such a cooling system would be powered by the battery 32.

To provide sufficient flexibility to allow the reservoir to move through the vasculature, the reservoir 12e may be divided into multiple reservoir segments separated by flexible connectors 74 similar to those described above for segmenting the device housing. In this embodiment, the pickup tube 18e preferably passes through each of the bladders 70, and extends to the pump 28e, which in this embodiment is positioned on an end of the device 10e.

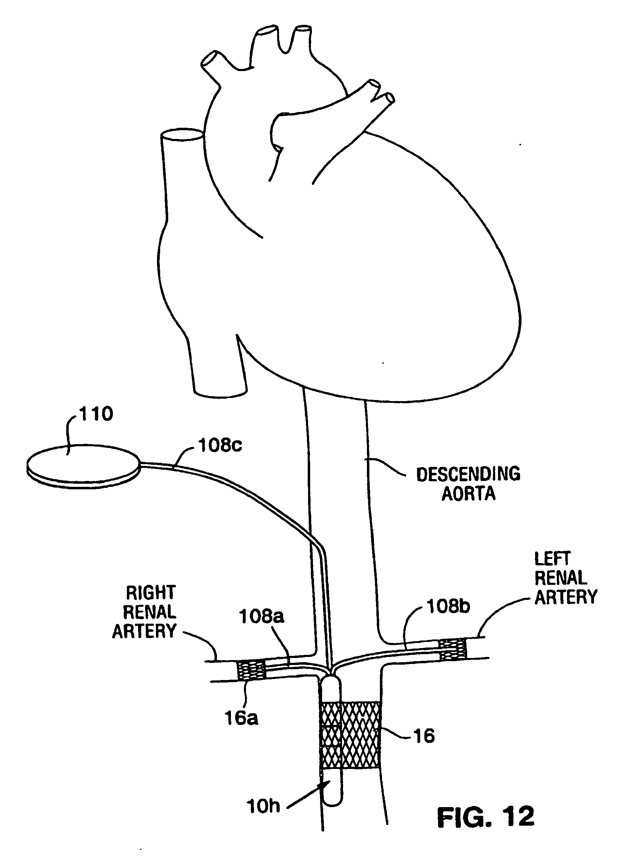

Although this description refers to the delivered agents as fluids, it should be mentioned that the reservoir and delivery mechanisms might be modified to allow agents in other forms such as agents in powder form (or lyophilized agents) to be used in place of liquid agents. For example, a powder form of an agent may be stored in a titanium reservoir within the device, and a delivery mechanism (for example the screw-auger type metering pump described in connection with Fig. 6) may feed quantities of the powder into a mixing chamber. A volume of saline or other fluid may be stored in a reservoir similar to the reservoir 12 of Fig. 12. A pump, such as any of the pumps described above, would then deliver a volume of the saline into the mixing chamber to mix with a quantity of the powder to form a liquid agent, which is then pumped or released into the blood stream. This embodiment allows a larger quantity of the agent to be stored within the device, and to remain stable within the body, than could be achieved by storing a liquid form of the agent within the device. The fluid reservoir could be refilled as needed using refill techniques described below. Other mechanisms for making use of powdered forms of agents might include delivering the agents directly into the bloodstream, or mixing the agents with blood drawn into the mixing chamber.

In another example, the delivery port of the device may be positioned to inject microspheres (e.g. agents embedded in a polymer matrix) loaded with the agent upstream of a vascularized bed or upstream of a vascularized tumor. The microspheres would embolize within small vessels in the vascularized bed or tumor, and over time would elute drug from the polymer matrix into the surrounding blood. Any of the pumps described above, including an auger-type metering pump or a piston-type pump, may be used to drive the microspheres into the bloodstream. The microspheres may be provided in a liquid solution, if desired. Agent embedded in micropheres can be advantageous in that it may allow the agent to remain stable within a reservoir within the body over extended periods of time.

It should also be noted at this point that the device might be configured to deliver multiple agents. According to this type of embodiment, the agents may be simultaneously released into the blood stream, independently released, or mixed in a mixing chamber within the device prior to release into the body. For example, the device may include one reservoir that stores a pharmaceutical agent in an inactivated state, and another reservoir that stores a chemical required to activate the agent. Combining the substances in a mixing chamber or simultaneously pumping them into the bloodstream activates the agent prior to or during administration.

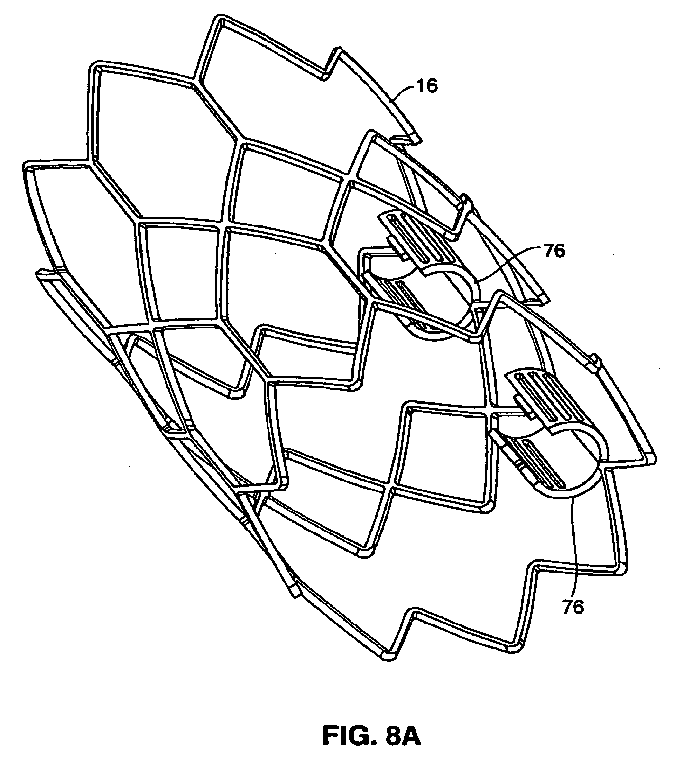

Referring again to Figs. 1 and 7, the intravascular drug delivery device preferably includes a retention mechanism 16 for retaining the device in the patient's vasculature. Although various means may be used to retain the device within the vasculature, one example of a retention device is the anchor 16 of the type shown in detail in Figs. 8A through 8E.

Referring to Fig. 8A, anchor 16 includes structural features that allow the anchor to radially engage a vessel wall. For example, a band, mesh or other framework formed of one or more shape memory (e.g., nickel titanium alloy, nitinol, thermally activated shape-memory material, or shape memory polymer) elements or stainless steel, Elgiloy, or MP35N elements may be used. The anchor may include anti-proliferative and anti-thrombogenic coatings, although in this embodiment he anchor structure 16 is preferably provided to promote tissue ingrowth to as to enhance anchor stability within the vessel. The anchor may also have drug delivery capability via a coating matrix impregnated with one or more pharmaceutical agents.

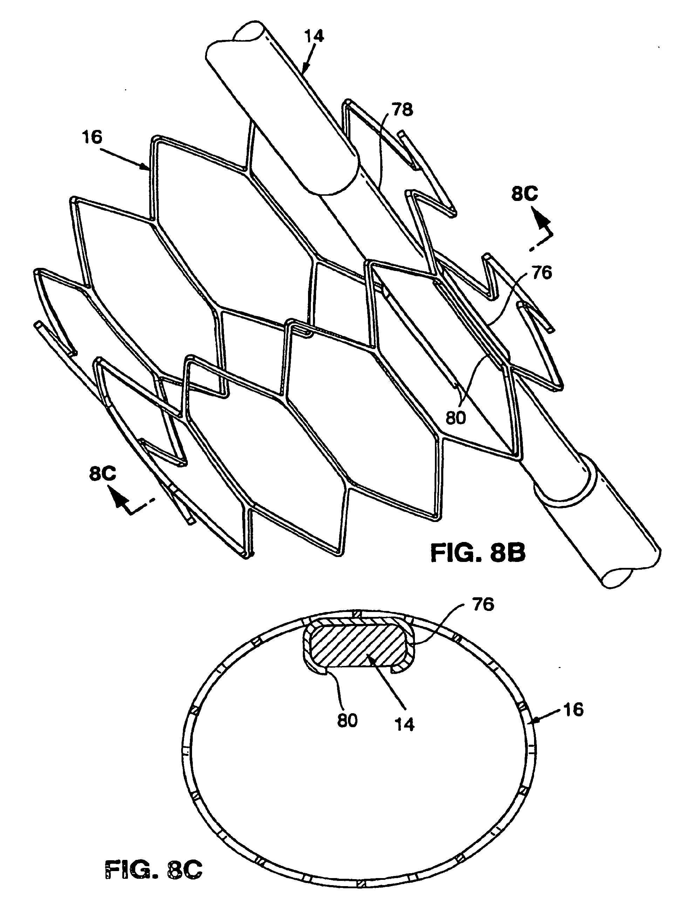

Fig. 8B shows one anchor 16 attached to a device body 14, although two or more such anchors may alternatively be used. As shown, anchor 16 is attached to the device body 14 by a collar 76, or other suitable connection. The body 14 may include a recessed portion 78 that allows the exterior of the anchor to sit flush with the exterior of the body when the anchor is its compressed position The recessed portion should have smooth contours in order to discourage thrombus formation on the device.

The anchor 16 and device body 14 may be detachably connected to the recessed portion using methods that allow the anchor 16 and the device 10 to be separated in situ, for permanent or temporary removal of the device 10. A detachable connection between the anchor 16 and device 10 may utilize a snap fit between the collar 76 and device body 14. As shown in Fig. 8C, both the collar 16 and the recessed portion 78 of the implant may include an elliptical cross-section. If it becomes necessary to remove the device 10 from the patient's body, the device may be torqued about its longitudinal axis, causing the device body 14 to cam the edges of the collar 76 to a slightly opened position, thereby allowing the device body to be passed between the edges 80 of the collar 76. In an alternative embodiment, a clevis pin-type connection may be made between the anchor 16 and the device body 14. Such a connection would be provided with a remotely actuated mechanism for releasing the clevis pin connection to thus permit separation of the device and the anchor.

The anchor may be configured such that the device 10 and anchor 16 share a longitudinal axis, or such that the axes of device 10 and anchor 16 are longitudinally offset.

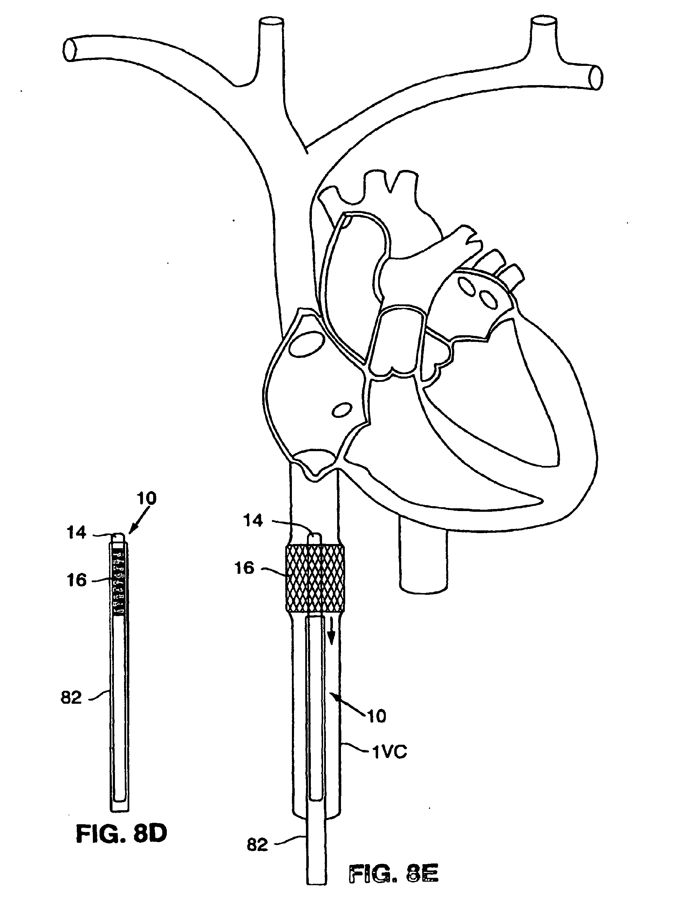

Referring to Fig. 8D, a retractable sheath 82 may be slidably positioned over the anchor 16 and device 10 so as to retain the anchor in its compressed position. Retraction of the sheath as indicated in Fig. 8E allows the anchor 16 to expand into contact with the surrounding walls of the vessel, thereby holding the medical implant in the desired location. Once deployed, the anchor 16 is preferably intimate to the vessel wall, which is distended slightly, allowing the vessel lumen to remain approximately continuous despite the presence of the anchor and thus minimizing turbulence or flow obstruction.

The anchor 16 is beneficial in that it is implanted integrally with the device, and thus does not require a separate implantation step. However, non-integral anchors may also be equally useful. Examples of non-integral anchors are described in U.S. Application No. 10/862,113, filed June 4, 2004, and entitled INTRAVASCULAR ELECTROPHYSIOLOGICAL SYSTEM AND METHODS.

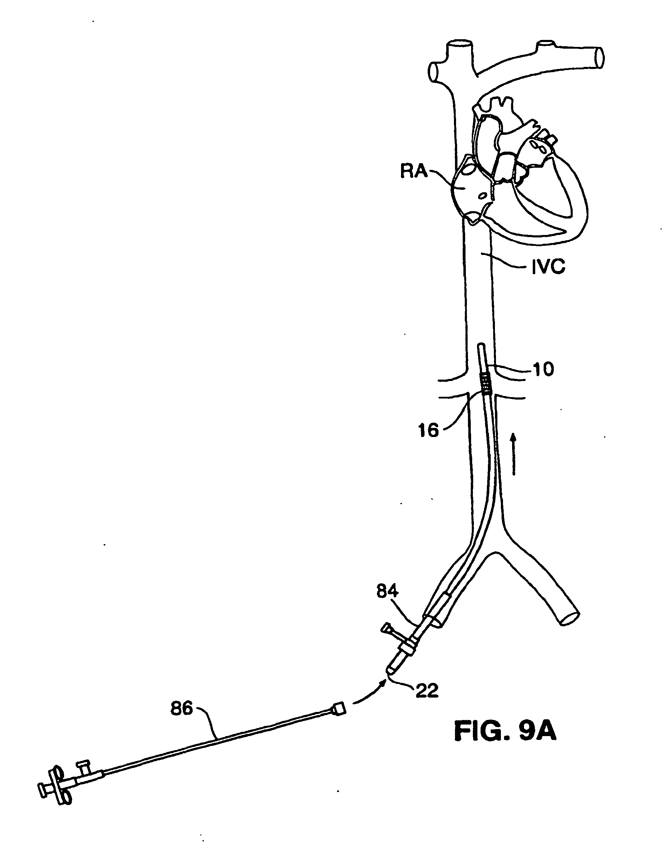

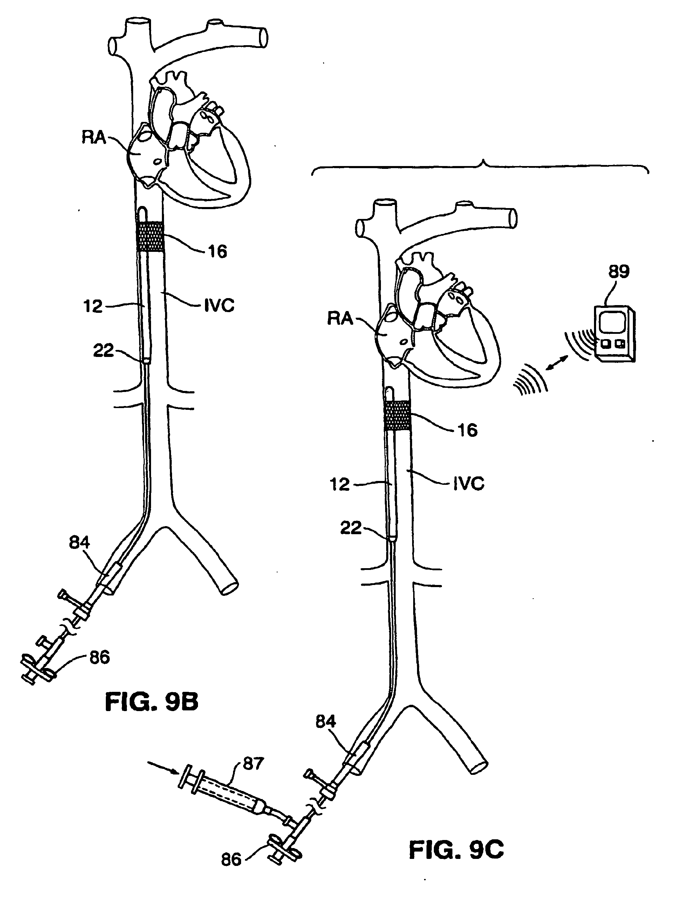

Figs. 9A through 9C illustrate a method for implanting an intravascular drug delivery device such as device 10 of Fig. 1 in the inferior vena cava (IVC). First, a small incision is formed in the femoral vein and an introducer 84 is inserted through the incision into the vein to keep the incision open during the procedure. Next, the device 10 is passed into the introducer 84, and pushed in a superior direction into the inferior vena cava ("IVC"). With an end of the device 10 still remaining outside the body, a mandrel 86 (Fig. 9B) is attached to the fill port 22 at the exposed end of the device 10. Pressure is applied against the mandrel 86 to advance the device 10 to the target location. See Fig. 9B.

Once the device has been properly positioned, the anchor 16 is deployed as described in connection with Figs. 8E and 8F. Although the anchor can be positioned anywhere along the length of the device, in one example the anchor position is positioned at a distance of approximately 3 -5 cm inferior to the right atrium (RA).

If the device 10 is not pre-filled with agent prior to implantation, agent may be introduced into the reservoir using the mandrel 86. Referring to Fig. 9C, a syringe 87 containing agent is coupled to the mandrel 86 and the agent is injected from the syringe 87 into the device reservoir 12 (Fig. 1) via the mandrel 86. The mandrel 86 and introducer 84 are removed from the body, leaving the device in place. The physician may next use an external telemetry unit 89 to set the dosing schedule and any other necessary parameters. Telemetry systems permitting external devices to communicate with implanted medical devices are known in the art. See, for example, U.S. Patent Nos. 6,824,561, 5,312,453 and 5,127,404. Alternatively, the device may be pre-programmed with a dosing schedule prior to implantation.

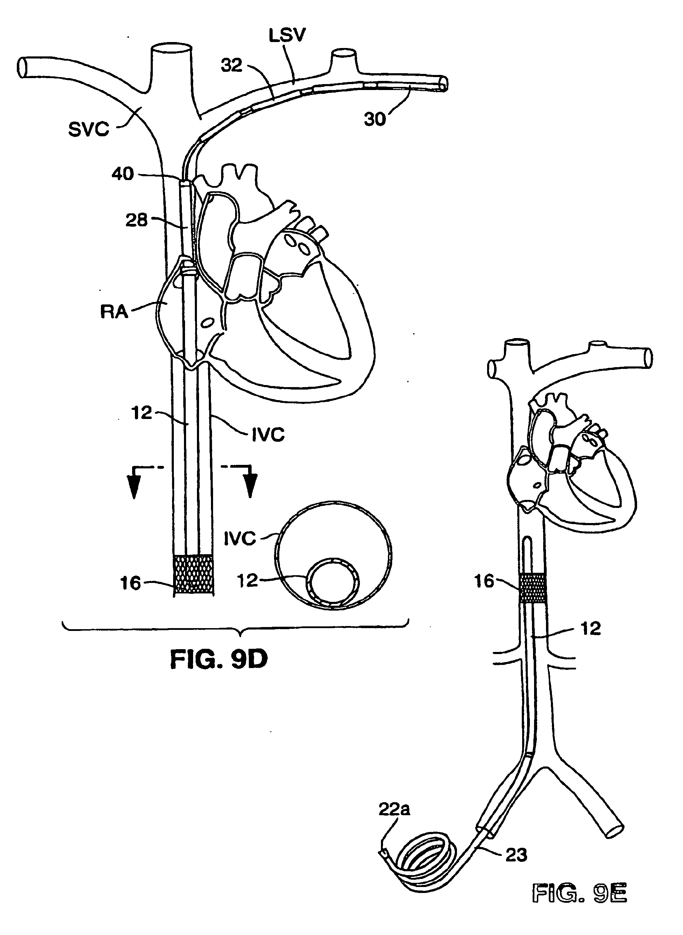

Referring to Fig. 9D, it should be noted that if mandated by the length of the device (due to, for example, a sizeable reservoir 12 and/or a string several batteries 32 as shown), the device may extend from the inferior vena cava (IVC), through the right atrium of the heart (RA), to the superior vena cava (SVC) and into the left subclavian vein (LSV) as shown. Implantation of a system of this length may be facilitated by the use of guidewires. Techniques for guidewire implantation of intravascular systems are shown and described in U.S. Application No. 10/862,113, filed June 4, 2004, and entitled INTRAVASCULAR ELECTROPHYSIOLOGICAL SYSTEM AND METHODS

Referring again to Fig. 9C, if at a later date it becomes necessary to add additional agent to the reservoir, the mandrel 86 may be reintroduced and coupled to the device 10 to allow agent to then be directed through the mandrel to the reservoir 12 using syringe 87 or similar means. To facilitate this process, the mandrel may have a distal coupling comprising a mouth that is significantly broader than the proximal end of the device. When the mandrel is advanced towards the device 10 within the vessel, the mouth will pass over the proximal end of the device 10 and will then be clamped over the proximal end of the device to sealingly engage the device to create a fluid coupling between the mandrel's fluid lumen and the fill port 22.

As another alternative shown in Fig. 9E, the device may include a permanent flexible refill tube 23 having fill port 22a at its proximal end. Tube 23 extends through the vasculature from the device location. The proximal end of the tube is positioned in a subcutaneous pocket and can be grasped and withdrawn through an incision (e.g. in the groin region) for fluid coupling to a refill vessel (e.g. syringe 87, Fig. 9C) outside the body. Once the reservoir 12 is refilled, tube 23 is tucked back into the pocket until accessed again during future refilling. Other methods for refilling the reservoir are discussed below.

Fig. 10 shows an alternative embodiment of an intravascular drug delivery device 10f utilizing an alternative arrangement of elements. The device is shown implanted within a blood vessel. In this arrangement, device 10 includes diagnostic electronics 88 for monitoring a physical and/or chemical condition of the patient, a valve 90, and valve control electronics 92 that are responsive to the diagnostic electronics to allow an appropriate dose of a drug compound to be released through the valve 90 and into the bloodstream. The drug compound is contained within a reservoir 94 which may, but need not be, similar to the reservoirs described elsewhere in this application. A refill port 96 allows the reservoir 94 to be refilled using a refill device passed through the vasculature. A mandrel connector 98 is engageable by an implantation mandrel 102 as described above. Although the refill port is shown as remote from the mandrel connection, the refill port may instead be positioned in fluid communication with the mandrel connector 98 as described in connection with the Fig. 1 embodiment so that the device can be refilled using mandrel 102.

The device may include a pressure generator 106 which osmotically generates sufficient pressure to drive agent from the reservoir and out of the valve 90. Alternatively, one of a variety of mechanisms, including those described above, may be used to drive agent from the reservoir.

A battery 106 powers the system and may be detachable from the device 12 for in-situ battery replacement. Mandrel connector 98 allows the mandrel 102 to be connected to the device and used for guiding the device (e.g., by pushing, pulling and/or torquing) through the patient's vasculature and into position during implantation. The connector 98 may take the form of a threaded bore for receiving a threaded screw member at the distal end of the mandrel 102, or it may have any other type of configuration for detachably engaging the distal end of the mandrel. As discussed, the mandrel may be used for refilling the reservoir in the device with pharmaceutical agents, and it also be used for in-situ replacement of the battery.

In yet another embodiment, the device may configured with a removeable drug reservoir, such that a mandrel may be used to separate the drug reservoir from the device 10 and to attach a fresh reservoir into the device.

Mandrel 102 may serve purely mechanical purposes, or it may also be a "smart mandrel" that provides electrical connections. Such connections can be used to couple the device (via an instrument cable) for direct electrical and/or electronic communication between the device and instrumentation located outside the body. This communication may be used several purposes, including device testing, initiation and/or programming during implantation, reaccess to the device for reprogramming or diagnostic testing, and/or recharging of the device battery.

The components within the device are disposed within an enclosure that may include some or all of the features of the housing described in connection with the Fig. 1 embodiment.

Device 10f may include delivery conduits such as elongate tubules 108 that create a fluid path from the device 10f to a target location within the body. For example, if it is desired to direct drugs to certain tissues or a particular organ, such as the brain, kidney or the heart, the distal ends of the delivery conduits 108 are passed through the appropriate vasculature to the target organ using guidewires or other implantation means.

Obviously, the positioning of the delivery conduits 108 will depend on the location to which drugs are to be delivered. For example, a delivery conduit 108 may extend into the left subclavian artery and may be positioned to administer drugs to the upper extremities, including the brain. As illustrated in Fig. 11, a device 10g may be positioned in the aorta and have a delivery conduit 108 positionable to direct agent into the hepatic artery (HA) or vessels further downstream of the hepatic artery for delivery of drugs to the liver for cancer treatment or for other purposes such as treatment of hepatitis. The vessel into which drug is delivered may be selected to be one that feeds the particular region of the organ of the liver to be treated, or one that feeds a vascularized tumor of the liver. The figure shows the delivery conduit 108 anchored in position using an (optional) anchor 16a. As yet another alternative shown in Fig. 12, the delivery conduits 108a,b may be positioned to deliver drugs into the blood flowing into the kidneys (e.g., for treatment of cardio-renal syndrome, cancer, or other conditions), with delivery conduit 108a extending into the right renal artery and delivery conduit 108b extending into the left renal artery. Agent flows into the delivery conduits 108a, 108b from the device 10h, which may be positioned in the descending aorta as shown.

Implantation of the Fig. 11 and 12 embodiments may be carried out using methods described above. However it should be noted that guidewire techniques may be used for implantation of delivery conduits 108, 108a, 108a (Figs. 11 and 12) in a manner similar to the lead implantation techniques described in U.S. Patent Application No. 10/862,113.

In the Fig. 11 and Fig. 12 embodiments it may be particularly useful to provide the agent in the form of microspheres (e.g. agent embedded in a polymer matrix), which once released onto the bloodstream would become lodged in capillaries, arterioles, or associated small blood vessels from which they would release the embedded agent over time. For example, referring again to the Fig. 11 embodiment, for cancer treatment the delivery conduit 108 may be selectively positioned to direct agent into a feeder vessel for a vascularized tumor of the liver, allowing highly selective control of microsphere delivery to a target site. One type of microsphere that may be useful for this purpose is the doxorubicin Drug Eluting Bead manufactured by Biocompatibles International PLC of Farnham, Surrey, UK.

The size of the microsphere may be selected to embolize within target feeder vessels, allowing the microsphere to limit blood flow to (and to thus starve) the tumor while simultaneously eluting agent into the tumor. The microspheres may be selected to be biologically activated, chemically activated, or activated through physical means. For example, biological activation may occur through bulk erosion of the polymer with consequent release of the drug, or through a diffusion of a more-rapidly dissolvable drug from a relatively less-rapidly degradable polymer matrix. Materials may be selected that are sensitive to particular conditions with the body (e.g. pH levels) so as to trigger agent release. As another example, chemical agents may be injected into contact with the microspheres (using the device 10 or a separate delivery mechanism) to trigger release of agent from the microspheres, or physical activation may be achieved by exposing the microspheres to energy generated by ultrasonic, magnetic, thermal or light sources. The microspheres may also be responsive to chemical de-activation, i.e. by injecting a chemical into contact with the microspheres to "tum-off" the microspheres thereby preventing further erosion or diffusion.

In an alternative embodiment, the system may be adapted to release radioactive beads (e.g. yttrium-90 impregnated glass beads) from the device to a targeted vascularized bed such as a tumor for cancer radiation treatment. Alternatively, a modified device containing radioactive particles (e.g. beads impregnated with Thulium 170 or P-32) may be advanced through the vasculature into a vessel within a tumor mass for radiation treatment. In this type of embodiment, a radio-protective storage housing might be positioned within the vasculature such that the modified device could be withdrawn into the radio-protective housing before and after the radiation treatment.

In some instances, it may be desirable to refill the system with additional agent. Thus, the embodiment of Fig. 12 (or the prior embodiments) may include an optional conduit 108c that is positioned to extend through a small needle stick formed in the aorta or other blood vessel. Conduit 108c is coupled to a subcutaneous reservoir or pad 110 for recharging/refilling the reservoir of device 10h. The subcutaneous reservoir 110 may be an elastomeric bladder positioned in the patient's body such as beneath the skin on the patient's chest. The membrane overlaying the bladder may be identified using a physical marker (e.g., tattoo) to allow the bladder to be easily located when it is necessary to recharge the reservoir. The subcutaneous reservoir 110 is recharged by injecting the bladder using a hypodermic needle containing the pharmaceutical compound. The bladder material is preferably self-resealing so that needle punctures will reseal following recharging. If necessary, the reservoir or pad and associated tubules may be coated or loaded with silver nitrate, steroids, anti-proliferative or anti-inflammatory compounds.

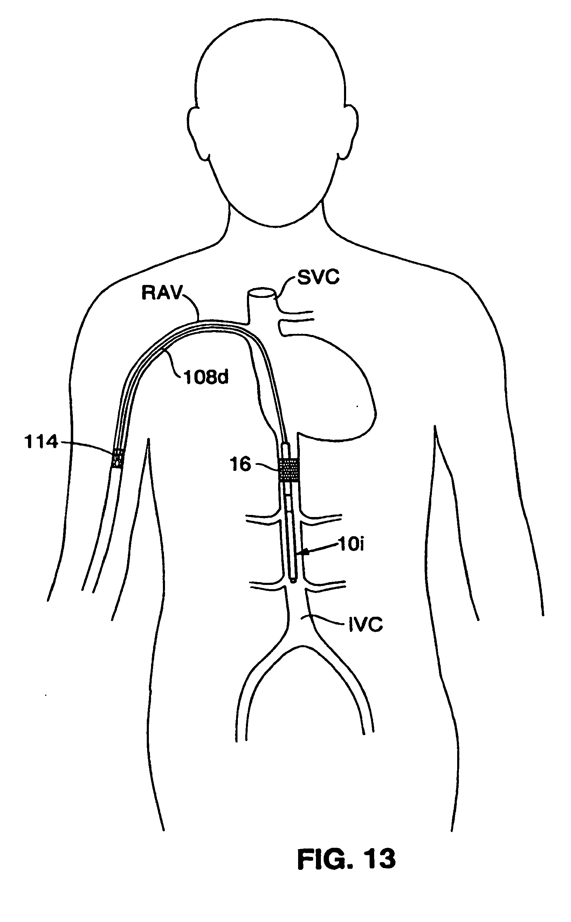

Fig. 13 shows a refill port 114 and conduit 108d for use with an implantable drug delivery device 10i. In this embodiment, the refill port 114 is positionable within a vessel that is easily accessible using a needle passed through the skin. One such vessel is the right axillary vein (RAV) accessible using a needle inserted into the arm. Another example is the femoral vein which may be accessed via a needle stick to the groin. Conduit 108d fluidly couples the refill port 114 to the device's agent reservoir. Refilling of the reservoir is carried out by locating the refill port 114 by palpating the skin or by locating a tatoo marking the port's location. A syringe containing the appropriate agent is then passed through the skin and into the port, and is used to inject the compound into the port. The compound flows through the conduit 108d and into the reservoir within the device 10i. The reservoir may be maintained at a negative pressure so as to draw the agent from the syringe once fluid communication is established. This provides feedback to the user that the syringe needle has been inserted at the proper location and can thus help to avoid injection of the agent directly into the patient in the event the refill port 114 is missed by the needle.

Various embodiments of systems and devices have been described herein. These embodiments are given only by way of example and are not intended to limit the scope of the present invention. It should be appreciated, moreover, that the various features of the embodiments that have been described might be combined in various ways to produce numerous additional embodiments. Moreover, while various materials, dimensions, shapes, implantation locations, etc have been described for use with disclosed embodiments, others besides those disclosed may be utilised without exceeding the scope of the invention. The following methods have also been described hereinbefore:
1. A method for delivering an agent into the bloodstream, comprising the steps of:
   providing an intravascular drug delivery system comprising an elongate flexible device body and a reservoir;
   percutaneously introducing the delivery system into a blood vessel and advancing the delivery system within the blood vessel, causing the device body to flex during movement through the blood vessel;
   anchoring the delivery system within the blood vessel;
   delivering an agent into the reservoir; and
   releasing agent from the reservoir into the bloodstream.
2. The method of paragraph 1 wherein the delivering step is carried out prior to the introducing step.
3. The method of paragraph 1 wherein the delivering step is carried out after the introducing step.
4. The method of paragraph 1 wherein the delivering step causes the reservoir to inflate.
5. The method of paragraph 1 wherein the device body includes a plurality of housing segments, and flexible regions interconnecting adjacent housing segments, and wherein the step of causing the device body to flex includes causing bending at the flexible regions.
6. The method of paragraph 1 wherein the releasing step includes driving agent into the bloodstream using a pump.
7. The method of paragraph 1 wherein:
   the providing step provides the system to include an elongate conduit extending from the device body; and
   the method includes positioning the conduit within a blood vessel; and
   the releasing step includes releasing agent through the conduit into the bloodstream.
8. The method of paragraph 7 wherein the anchoring step includes anchoring the delivery system with the device body in a first blood vessel and positioning at least a portion of the conduit in a second blood vessel different from the first blood vessel.
9. The method of paragraph 8 wherein the second blood vessel is a hepatic artery.
10. The method of paragraph 8 wherein the second blood vessel is a renal artery.
11. The method of paragraph 8 wherein the second blood vessel is a subclavian artery.
12. The method of paragraph 1 wherein:
   the device body and reservoir include a mixing chamber, a first chamber containing a first substance, and a second chamber containing a second substance; and
   the method includes the steps of directing a quantity of first substance from the first chamber into the mixing chamber, and directing a quantity of second substance from the second chamber into the mixing chamber to form the agent; and
   the releasing step includes releasing the agent from the mixing chamber.
13. The method according to paragraph 12 wherein the first substance is a liquid and the second substance is a powder.
14. The method according to paragraph 12 wherein the step of directing a quantity of second substance into the mixing chamber includes the step of activating a metering pump disposed in the second chamber.
15. The method according to paragraph 12 wherein the first chamber is an inflatable bladder.
16. The method according to paragraph 1 wherein the step of delivering the agent includes delivering microspheres containing the agent.
17. The method according to paragraph 16 wherein releasing step includes releasing the microspheres from the reservoir.
18. The method according to paragraph 17 wherein the method further includes causing the microspheres to embolize within a vessel and allowing agent to elute from the microspheres into the bloodstream.
19. The method according to paragraph 1, wherein the system includes a pump and a controller within the device body and wherein in the releasing step the controLler causes the pump to pump agent into the bloodstream according to a dosing schedule.
20. The method according to paragraph 19 further including the step of programming the dosing schedule into the controller prior to the introducing step.
21. The method according to paragraph 19 further including the step of programming the dosing schedule into the controller after the introducing step.
22. The method according to paragraph 21 wherein the introducing step includes the step of attaching a delivery mandrel to the device body and pushing the mandrel to position the device body within the blood vessel, and wherein the programming step includes the step of electronically coupling the delivery mandrel to a programming device positioned outside the body and transmitting the dosing schedule to the controller using the programming device.
23. The method according to paragraph 21 wherein the controller includes telemetry circuitry and the system further includes a remote communication device operable from outside the body to communicate with the telemetry circuitry, and wherein the programming step includes transmitting the dosing schedule to the controller using the remote communication device.
24. The method according to paragraph 19 wherein the system includes a sensor on the device body, wherein the method includes the step of detecting a parameter within the body using the sensor, and wherein in the releasing step the controller is responsive to a parameter detected by the sensor to cause agent to be pumped into the bloodstream.
25. The method according to paragraph 24 wherein the detecting step determines a degree of the detected parameter, and wherein in the releasing step the controller is responsive to the detected degree to determine a dosing schedule and to cause the agent to be pumped according to the determined dosing schedule.
26. The method according to paragraph 24 wherein the sensor detects a physical or chemical parameter.
27. The method according to paragraph 26 wherein the sensor detects a parameter from the group of parameters consisting of arterial pressure, cardiac output, heart rate, Q-T interval, AVO₂ difference, blood pH, blood gas levels, blood sugar, and biochemical markers.
28. The method according to paragraph 9 wherein the step of delivering the agent includes delivering microspheres containing the agent and wherein the releasing step includes releasing the microspheres from the reservoir, and wherein the method further includes causing the microspheres to embolize within a vessel and allowing agent to elute from the microspheres into the bloodstream.
29. The method according to paragraph 28 wherein the microspheres include a chemotherapeutic agent.

## Claims

1. A system for drug delivery, the system comprising
An implantable reservoir (12) and an implantable elongate device body (14), wherein the reservoir (12) and the device body (14) are ptoportioned for implantation within a blood vessel, the elongate device body (14) is flexible and a pump (28) is housed within the device body (14) and fluidly coupled to the reservoir (12) and operable to direct agent from the reservoir into the bloodstream **characterised in that** said elangate device body has an anchor (16) coupled thereto and expandable into contact with a wall of the blood vessel.

2. The system according to claim 1 wherein the reservoir (12) is housed within the device body (14).

3. The system according to claim 2 wherein the reservoir comprises an inflatable bladder disposed within the device body.

4. The system according to claim 1 wherein the reservoir (12) is external to the device body (14).

5. The system according to claim 4 wherein the reservoir comprises an elongate inflatable bladder extending longitudinally from the device body.

6. The system according to claim 1, wherein the pump includes a motor (26) and an energy source (32) electrically coupled to the motor.

7. The system according to claim 5 wherein the energy source includes a battery (32).

8. The system according to claim 1 wherein the reservoir (12) extends longitudinally from the device body.

9. The system according to claim 1 wherein the device body (14) includes at least one flexible region (24), the region sufficiently flexible to permit bending of the device body during movement of the device through a blood vessel.

10. The system of claim 9, wherein the device body (14) includes a plurality of housing segments (25a, 25b, 25c), and flexible regions (24) interconnecting adjacent housing segments.

11. The system of claim 10 wherein the pump (14) is sealed within one of the housing segments (25a), and wherein the system further includes a motor (26) and a battery (32) each sealed within housing segments (25a, 25c).

12. The system of claim 10, wherein the reservoir (12e) is positioned within a plurality of the housing segments (72).

13. The system of claim 12 wherein the reservoir (12e) includes a plurality of bladders (70), each positioned in a corresponding one of the housing segments (72).

14. The system of claim 1 wherein the device body (14) is formed of titanium.

15. The system of claim 1 wherein the device body (14) has a length of approximately 10cm or greater.

16. The system of claim 1 wherein the device body (14) has a cross-sectional area of approximately 79 mm² or less.

17. The system of claim 16, wherein the device body (14) has a cross-sectional area of approximately 40 mm² or less.

18. The system of claim 1 wherein the pump (28) is selected from the group of pumps consisting of gear pumps, peristaltic pumps, solenoid pumps, vacuum pumps, venturi pumps, double-acting membrane pumps, metering pumps, syringe pumps and vaporisation displacement pumps.

19. The system according to claim 1 wherein the reservoir (12) contains microspheres containing an agent.

20. The system of claim 1, further including telemetry circuitry within the device housing and a remote communication device (89) operable from outside the body to communicate with the telemetry circuitry.

21. The system according to claim 1 further including at least one elongate conduit (108) extending from the device body, wherein the pump is operable to direct agent through the conduit into the bloodstream.

22. The system according to claim 21, further including an anchor (16a) expandable to anchor the elongate conduit (108) within a blood vessel.

23. The system according to claim 21 wherein the device body (14) is positionable in a first blood vessel and the conduit (108) is positionable in a second, different, blood vessel.

24. The system according to claim 1 wherein the system includes a fill conduit (23) extending from the reservoir (12), a fill port (22) coupled to the fill conduit (23), and an extracorporeal reservoir connectable to the fill conduit.

25. The system according to claim 24 wherein the fill port (114) is positionable within a blood vessel.

26. The system according to claim 24 wherein the fill port (110) is positionable within a subcutaneous pocket.

27. The system according to claim 24 wherein the extracorporeal reservoir comprises a syringe.

28. The system according to claim 24 wherein the syringe includes a needle engageable with the fill port.

29. The system according to claim 1 wherein:
the reservoir includes a mixing chamber, a first chamber containing a first substance, and a second chamber containing a second substance;
the pump is a dispensing pump positioned to pump agent from the mixing chamber into the bloodstream;
the second system further includes a first pump for pumping the first substance from the first chamber into the mixing chamber;
the second system further includes a second pump for pumping the second substance from the second chamber into the mixing chamber.

30. The system according to claim 29 wherein the first substance is a liquid and the second substance is a powder.

31. The system according to claim 30 wherein the second pump is a metering pump.

32. The system according to claim 30 wherein the first chamber is an inflatable bladder.

33. The system according to claim 32 wherein the inflatable bladder is positioned external to the device body, and wherein the second chamber is positioned within the device body.

34. The system according to claim 1, further including:
a controller (30) within the device body (14), wherein the pump is controlled by the controller to pump agent into the bloodstream.

35. The system according to claim 34 wherein the controller is programmed to cause agent delivery according to a predetermined dosing schedule.

36. The system according to claim 34, further including a sensor on the device body for detecting a condition within the patient's body, wherein the controller is responsive to a condition detected by the sensor to cause agent to be pumped into the bloodstream.

37. The system according to claim 36 wherein the controller is responsive to a degree of the condition detected by the sensor to determine a dosage of agent to be pumped into the bloodstream.

38. The system according to claim 34 wherein the controller includes telemetry circuitry and the system includes a remote communication device operable from outside the body to communicate with the telemetry circuitry, wherein the controller is responsive to signals received by the telemetry circuitry to cause agent to be pumped into the bloodstream according to a specified dosing schedule.

39. The system according to claim 1 wherein the reservoir contains radioactive particles.

## Patentansprüche

1. System zur Arzneimittelabgabe, wobei das System Folgendes umfasst:
einen implantierbaren Vorratsbehälter (12) und einen implantierbaren länglichen Gerätekörper (14), wobei der Vorratsbehälter (12) und der Gerätekörper (14) zur Implantation in ein Blutgefäß proportioniert sind, der längliche Gerätekörper (14) flexibel ist und eine Pumpe (28) in dem Gerätekörper (14) eingehäust und mit dem Vorratsbehälter (12) strömungsverbunden ist und betreibbar ist, um ein Agens aus dem Vorratsbehälter in den Blutstrom zu führen, wobei der genannte längliche Gerätekörper einen Anker (16) hat, der mit ihm gekoppelt ist und zum Kontakt mit einer Wand des Blutgefäßes ausdehnbar ist.

2. System nach Anspruch 1, wobei der Vorratsbehälter (12) in dem Gerätekörper (14) eingehäust ist.

3. System nach Anspruch 2, wobei der Vorratsbehälter eine aufblasbare Blase umfasst, die in dem Gerätekörper angeordnet ist.

4. System nach Anspruch 1, wobei sich der Vorratsbehälter (12) außerhalb des Gerätekörpers (14) befindet.

5. System nach Anspruch 4, wobei der Vorratsbehälter eine längliche aufblasbare Blase umfasst, die sich längs vom Gerätekörper erstreckt.

6. System nach Anspruch 1, wobei die Pumpe einen Motor (26) und eine elektrisch mit dem Motor gekoppelte Energiequelle (32) beinhaltet.

7. System nach Anspruch 5, wobei die Energiequelle eine Batterie (32) beinhaltet.

8. System nach Anspruch 1, wobei der Vorratsbehälter (12) sich längs vom Gerätekörper aberstreckt.

9. System nach Anspruch 1, wobei der Gerätekörper (14) wenigstens eine flexible Region (24) beinhaltet, wobei die Region ausreichend flexibel ist, um das Biegen des Gerätekörpers während der Bewegung des Geräts durch ein Blutgefäß zuzulassen.

10. System nach Anspruch 9, wobei der Gerätekörper (14) eine Vielzahl von Gehäusesegmenten (25a, 25b, 25c) und flexiblen Regionen (24), die benachbarte Gehäusesegmente miteinander verbinden, beinhaltet.

11. System nach Anspruch 10, wobei die Pumpe (14) in einem der Gehäusesegmente (25a) eingekapselt ist und wobei das System ferner einen Motor (26) und eine Batterie (32) beinhaltet, die jeweils in Gehäusesegmenten (25a, 25c) eingekapselt sind.

12. System nach Anspruch 10, wobei der Vorratsbehälter (12e) in einer Vielzahl der Gehäusesegmente (72) positioniert ist.

13. System nach Anspruch 12, wobei der Vorratsbehälter (12e) eine Vielzahl von Blasen (70) beinhaltet, die jeweils in einem entsprechenden der Gehäusesegmente (72) positioniert sind.

14. System nach Anspruch 1, wobei der Gerätekörper (14) aus Titan hergestellt ist.

15. System nach Anspruch 1, wobei der Gerätekörper (14) eine Länge von etwa 10 cm oder mehr hat.

16. System nach Anspruch 1, wobei der Gerätekörper (14) eine Querschnittsfläche von etwa 79 mm² oder weniger hat.

17. System nach Anspruch 16, wobei der Gerätekörper (14) eine Querschnittsfläche von etwa 40 mm² oder weniger hat.

18. System nach Anspruch 1, wobei die Pumpe (28) aus der Gruppe von Pumpen gewählt ist, die Zahnradpumpen, peristaltische Pumpen, elektromagnetische Pumpen, Vakuumpumpen, Strahlpumpen, doppeltwirkende Membranpumpen, Dosierpumpen, Spritzenpumpen und Zerstäuberverdrängerpumpen besteht.

19. System nach Anspruch 1, wobei der Vorratsbehälter (12) ein Agens enthaltende Mikrokügelchen enthält.

20. System nach Anspruch 1, das ferner eine Telemetrieschaltung in dem Gerätegehäuse und eine abgesetzte Kommunikationsvorrichtung (98) beinhaltet, die von außerhalb des Körpers bedient werden kann, um mit der Telemetrieschaltung zu kommunizieren.

21. System nach Anspruch 1, das ferner wenigstens eine längliche Leitung (108) beinhaltet, die sich von dem Gerätekörper erstreckt, wobei die Pumpe betreibbar ist, um das Agens durch die Leitung in den Blutstrom zu führen.

22. System nach Anspruch 21, das ferner einen Anker (16a) beinhaltet, der zur Verankerung der länglichen Leitung (108) in einem Blutgefäß ausdehnbar ist.

23. System nach Anspruch 21, wobei der Gerätekörper (14) in einem ersten Blutgefäß positioniert werden kann und die Leitung (108) in einem zweiten, anderen Blutgefäß positioniert werden kann.

24. System nach Anspruch 1, wobei das System eine Füllleitung (23), die sich von dem Vorratsbehälter (12) erstreckt, einen Füllport (22), der mit der Füllleitung (23) gekoppelt ist, und einen extrakorporalen Vorratsbehälter, der mit der Füllleitung verbunden werden kann, beinhaltet.

25. System nach Anspruch 24, wobei der Füllport (114) in einem Blutgefäß positioniert werden kann.

26. System nach Anspruch 24, wobei der Füllport (110) in einer subkutanen Tasche positioniert werden kann.

27. System nach Anspruch 24, wobei der extrakorporale Vorratsbehälter eine Spritze umfasst.

28. System nach Anspruch 24, wobei die Spritze eine Nadel beinhaltet, die mit dem Füllport in Eingriff gebracht werden kann.

29. System nach Anspruch 1, wobei:
der Vorratsbehälter eine Mischkammer, eine erste Kammer, die eine erste Substanz enthält, und eine zweite Kammer, die eine zweite Substanz enthält, beinhaltet,
die Pumpe eine Abgabepumpe ist, die zum Fördern von Agens aus der Mischkammer in den Blutstrom positioniert ist,
das System ferner eine erste Pumpe zum Pumpen der ersten Substanz von der ersten Kammer in die Mischkammer beinhaltet,
das System ferner eine zweite Pumpe zum Pumpen der zweiten Substanz von der zweiten Kammer in die Mischkammer beinhaltet.

30. System nach Anspruch 29, wobei die erste Substanz eine Flüssigkeit ist und die zweite Substanz ein Pulver ist.

31. System nach Anspruch 30, wobei die zweite Pumpe eine Dosierpumpe ist.

32. System nach Anspruch 30, wobei die erste Kammer eine aufblasbare Blase ist.

33. System nach Anspruch 32, wobei die aufblasbare Blase außerhalb des Gerätekörpers positioniert ist und wobei die zweite Kammer im Gerätekörper positioniert ist.

34. System nach Anspruch 1, das ferner Folgendes beinhaltet:
einen Controller (30) im Gerätekörper (14), wobei die Pumpe von dem Controller zum Pumpen des Agens in den Blutstrom gesteuert wird.

35. System nach Anspruch 34, wobei der Controller so programmiert ist, dass er die Agensabgabe gemäß einem vorbestimmten Dosierungsplan veranlasst.

36. System nach Anspruch 34, das ferner einen Sensor an dem Gerätekörper zum Erkennen eines Zustands in dem Körper des Patienten beinhaltet, wobei der Controller auf einen von dem Sensor erkannten Zustand reagiert, um zu veranlassen, dass Agens in den Blutstrom gefördert wird.

37. System nach Anspruch 36, wobei der Controller auf einen Grad des von dem Sensor erkannten Zustands reagiert, um eine in den Blutstrom zu pumpende Agensdosis zu ermitteln.

38. System nach Anspruch 34, wobei der Controller eine Telemetrieschaltung beinhaltet und das System eine abgesetzte Kommunikationsvorrichtung beinhaltet, die von außerhalb des Körpers bedient werden kann, um mit der Telemetrieschaltung zu kommunizieren, wobei der Controller auf von der Telemetrieschaltung empfangene Signale reagiert, um zu veranlassen, dass Agens gemäß einem spezifizierten Dosierungsplan in den Blutstrom gefördert wird.

39. System nach Anspruch 1, wobei der Vorratsbehälter radioaktive Teilchen enthält.

## Revendications

1. Système d'administration de médicament, le système comprenant :
un réservoir implantable (12) et un corps de dispositif allongé implantable (14), le réservoir (12) et le corps de dispositif (14) étant proportionnés pour être implantés dans un vaisseau sanguin, le corps de dispositif allongé (14) étant souple et une pompe (28) étant logée dans le corps de dispositif (14) et couplée fluidiquement au réservoir (12) et actionnable pour diriger un agent depuis le réservoir jusque dans le système sanguin, **caractérisé en ce que** ledit corps de dispositif allongé comporte un ancrage (16) couplé à celui-ci et extensible pour être mis en contact avec une paroi du vaisseau sanguin.

2. Système selon la revendication 1, dans lequel le réservoir (12) est logé à l'intérieur du corps de dispositif (14).

3. Système selon la revendication 2, dans lequel le réservoir comprend une vessie gonflable disposée à l'intérieur du corps de dispositif.

4. Système selon la revendication 1, dans lequel le réservoir (12) est externe au corps de dispositif (14).

5. Système selon la revendication 4, dans lequel le réservoir comprend une vessie gonflable allongée qui s'étend longitudinalement depuis le corps de dispositif.

6. Système selon la revendication 1, dans lequel la pompe comporte un moteur (26) et une source d'énergie (32) couplée électriquement au moteur.

7. Système selon la revendication 5, dans lequel la source d'énergie comporte une batterie (32).

8. Système selon la revendication 1, dans lequel le réservoir (12) s'étend longitudinalement depuis le corps de dispositif.

9. Système selon la revendication 1, dans lequel le corps de dispositif (14) comporte au moins une région souple (24), la région étant suffisamment souple pour permettre la flexion du corps de dispositif durant le mouvement du dispositif à travers un vaisseau sanguin.

10. Système selon la revendication 1, dans lequel le corps de dispositif (14) comporte une pluralité de segments de logement (25a, 25b, 25c), et des régions souples (24) qui interconnectent des segments de logement adjacents.

11. Système selon la revendication 10, dans lequel la pompe (14) est scellée à l'intérieur de l'un des segments de logement (25a), et dans lequel le système comporte en outre un moteur (26) et une batterie (32) scellés chacun dans des segments de logement (25a, 25c).

12. Système selon la revendication 10, dans lequel le réservoir (12e) est positionné à l'intérieur d'une pluralité des segments de logement (72).

13. Système selon la revendication 12, dans lequel le réservoir (12e) comporte une pluralité de vessies (70), positionnées chacune dans un segment correspondant des segments de logement (72).

14. Système selon la revendication 1, dans lequel le corps de dispositif (14) est formé de titane.

15. Système selon la revendication 1, dans lequel le corps de dispositif (14) a une longueur d'approximativement 10 cm ou plus.

16. Système selon la revendication 1, dans lequel le corps de dispositif (14) a une superficie en coupe d'approximativement 79 mm² ou moins.

17. Système selon la revendication 16, dans lequel le corps de dispositif (14) a une superficie en coupe d'approximativement 40 mm² ou moins.

18. Système selon la revendication 1, dans lequel la pompe (28) est sélectionnée dans le groupe de pompes consistant en pompes à engrenage, pompes péristaltiques, pompes à solénoïde, pompes à vide, pompes venturi, pompes à membrane à double effet, pompes de dosage, pompes à seringue et pompes volumétriques de vaporisation.

19. Système selon la revendication 1, dans lequel le réservoir (12) contient des microsphères qui renferment un agent.

20. Système selon la revendication 1, comportant en outre des circuits de télémesure à l'intérieur du logement de dispositif et un dispositif de communication à distance (89) actionnable depuis l'extérieur du corps afin de communiquer avec les circuits de télémesure.

21. Système selon la revendication 1, comportant en outre au moins un conduit allongé (108) s'étendant depuis le corps de dispositif, la pompe étant actionnable pour diriger l'agent à travers le conduit jusque dans le système sanguin.

22. Système selon la revendication 21, comportant en outre un ancrage (16a) extensible pour ancrer le conduit allongé (108) à l'intérieur d'un vaisseau sanguin.

23. Système selon la revendication 21, dans lequel le corps de dispositif (14) est positionnable dans un premier vaisseau sanguin et le conduit (108) est positionnable dans un second vaisseau sanguin différent.

24. Système selon la revendication 1, comportant un conduit de remplissage (23) s'étendant depuis le réservoir (12), un point de remplissage (22) couplé au conduit de remplissage (23), et un réservoir extracorporel pouvant être connecté au conduit de remplissage.

25. Système selon la revendication 24, dans lequel l'orifice de remplissage (114) est positionnable dans un vaisseau sanguin.

26. Système selon la revendication 24, dans lequel l'orifice de remplissage (110) est positionnable à l'intérieur d'une poche sous-cutanée.

27. Système selon la revendication 24, dans lequel le réservoir extra-corporel comprend une seringue.

28. Système selon la revendication 24, dans lequel la seringue comporte une aiguille engageable avec l'orifice de remplissage.

29. Système selon la revendication 1, dans lequel :
le réservoir comporte une chambre de mixage, une première chambre contenant une première substance, et une seconde chambre contenant une seconde substance ;
la pompe est une pompe d'administration positionnée pour pomper un agent depuis la chambre de mixage jusque dans le système sanguin ;
le système comportant en outre une première pompe pour pomper la première substance depuis la première chambre jusque dans la chambre de mixage ;
le système comportant en outre une seconde pompe pour pomper la seconde substance depuis la seconde chambre jusque dans la chambre de mixage.

30. Système selon la revendication 29, dans lequel la première substance est un liquide et la seconde substance est une poudre.

31. Système selon la revendication 30, dans lequel la seconde pompe est une pompe de dosage.

32. Système selon la revendication 30, dans lequel la première chambre est une vessie gonflable.

33. Système selon la revendication 32, dans lequel la vessie gonflable est positionnée à l'extérieur du corps du dispositif, et dans lequel la seconde chambre est positionnée à l'intérieur du corps de dispositif.

34. Système selon la revendication 1, comportant en outre :
une unité de commande (30) à l'intérieur du corps de dispositif (14), la pompe étant commandée par l'unité de commande pour pomper l'agent dans le système sanguin.

35. Système selon la revendication 34, dans lequel l'unité de commande est programmée pour entraîner la distribution de l'agent en fonction d'un programme de dosage prédéterminé.

36. Système selon la revendication 34, comportant en outre un capteur sur le corps de dispositif afin de détecter une condition dans le corps du patient, l'unité de commande étant sensible à une condition détectée par le capteur pour déclencher le pompage de l'agent dans le système sanguin.

37. Système selon la revendication 36, dans lequel l'unité de commande est sensible à un certain degré de la condition détectée par le capteur pour déterminer un dosage de l'agent à pomper dans le système sanguin.

38. Système selon la revendication 34, dans lequel l'unité de commande comporte des circuits de télémesure et le système comporte un dispositif de communication à distance actionnable depuis l'extérieur du corps pour communiquer avec les circuits de télémesure, l'unité de commande étant sensible à des signaux reçus par les circuits de télémesure pour déclencher le pompage de l'agent dans le système sanguin en fonction d'un programme de dosage spécifié.

39. Système selon la revendication 1, dans lequel le réservoir contient des particules radioactives.
